(19) Europäisches Patentamt
European Patent Office
Office européen des brevets

(11) **EP 3 185 691 B1**

(12) **EUROPEAN PATENT SPECIFICATION**

(45) Date of publication and mention
of the grant of the patent:
**24.04.2019 Bulletin 2019/17**

(21) Application number: **15741208.1**

(22) Date of filing: **23.07.2015**

(51) Int Cl.:
**A23F 5/04** $^{(2006.01)}$

(86) International application number:
**PCT/EP2015/066923**

(87) International publication number:
**WO 2016/016101 (04.02.2016 Gazette 2016/05)**

(54) **ROASTED LAURINA COFFEE, METHOD FOR ITS PREPARATION, AND USE FOR PREVENTING METABOLIC SYNDROME**

LAURINA RÖSTKAFFEE, VERFAHREN ZUR DESSEN HERSTELLUNG, UND DEREN VERWENDUNG ZUR VORBEUGUNG DES STOFFWECHSELSYNDROMS

CAFÉ TORRÉFIÉ LAURINA, PROCÉDÉ POUR SON PRÉPARATION, ET SON UTILISATION POUR PRÉVENIR LE SYNDROME MÉTABOLIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.08.2014 IT MI20141412**

(43) Date of publication of application:
**05.07.2017 Bulletin 2017/27**

(73) Proprietor: **Illycaffe' S.p.A.**
**34147 Trieste (IT)**

(72) Inventors:
• **NAVARINI, Luciano**
**34100 Trieste (IT)**
• **COLOMBAN, Silvia**
**34100 Trieste (IT)**
• **SUGGI LIVERANI, Furio**
**34123 Trieste (IT)**
• **MAZZUCCO, Sara**
**32013 Longarone (IT)**

• **BIOLO, Gianni**
**34100 Trieste (IT)**

(74) Representative: **Modiano, Micaela Nadia et al**
**Modiano & Partners**
**Via Meravigli, 16**
**20123 Milano (IT)**

(56) References cited:
**EP-A1- 0 606 759       EP-A1- 1 808 078**
**WO-A1-2014/048888     US-A1- 2010 112 098**

• **TAGUCHI H ET AL: "Trigonelline Content in Coffee Beans and the Thermal Conversion of Trigonelline into Nicotinic Acid during the Roasting of Coffee Beans", AGRICULTURAL AND BIOLOGICAL CHEMISTRY, JAPAN SOC. FOR BIOSCIENCE, BIOTECHNOLOGY AND AGROCHEM, TOKYO, JP, vol. 49, no. 12, 1 January 1985 (1985-01-01), pages 3467-3471, XP002998884, ISSN: 0002-1369**

**Description**

The present invention relates to a roasted coffee, beverages prepared with said roasted coffee, a method for preparing said roasted coffee and its use in the prevention of type 2 diabetes.

Infusions of coffee are the most commonly drunk beverages worldwide. Various studies of the links between drinking coffee and health have recently cast light on new information about the link between drinking this beverage and the risk of developing type 2 diabetes.

Diabetes is the fourth-highest cause of death in industrialized countries, and type 2 diabetes is its most common form, accounting for around 90% of cases. Furthermore, diabetes is often associated with the development of other metabolic anomalies (for example, dyslipidemia, abdominal obesity, activation of low-grade systemic inflammation, hypertension), thus representing a major risk factor for cardiovascular diseases (Huxley et al., British Medical Journal, 2006; 332:73-78) . In fact, the cardiovascular events represent the leading cause of premature death in diabetic patients (Morrish et al., Diabetologia, 2001; 44(Suppl. 2):S14-21).

The lifestyle of the subjects, in particular with regard to their diet and their level of physical activity, plays a central role in the development of type 2 diabetes. Recent studies have led to the observation that a moderate and prolonged consumption of coffee significantly reduces the risk of developing type 2 diabetes (Tuomilehto et al., JAMA. 2004; 291:1213-1219; and Sartorelli et al., Am J Clin Nutr., 2010; 91:1002-1012).

It is interesting to note that even people who drink decaffeinated coffee have been observed to have a reduction of the risk of developing type 2 diabetes. In particular, the risk is approximately one-third lower in persons who consume 3-4 cups of decaffeinated coffee per day, compared to persons who do not drink coffee (Huxley et al., Arch Intern Med., 2009; 169:2053-2063).

These results therefore suggest that the protective effect of coffee against the development of type 2 diabetes is not directly related to the caffeine content in the beverage. Indeed, in addition to caffeine, which effectively accounts for only around 2% of the chemical profile, coffee contains a further large number of bioactive molecules (over 1,000 volatile and non-volatile compounds have been identified in roasted coffee), including lipids, polysaccharides, phenolic compounds, melanoidin, soluble fiber, and minerals.

As is known, oxidative stress is recognized to have a key role in the development of insulin resistance, and thus of type 2 diabetes. Coffee is a rich source of phenolic compounds with antioxidant action (the total content ranges from 200 mg to 550 mg per cup) and, owing to its enormous consumption worldwide, such beverage can thus be considered one of the main sources of antioxidants in the diet in many countries (Halvorsen et al. , Am J Clin Nutr. 2006, 84:95-135; and Pellegrini et al., J Nutr. 2003, 133:2812-2819).

The development of systemic inflammation, even if low grade, is another key process in the pathogenesis of type 2 diabetes. Several studies have found the existence of an inverse relationship between the consumption of coffee and the activation of systemic inflammation phenomena and endothelial dysfunction in healthy subjects (Schulze et al., Am J Clin Nutr., 2005, 82:675-684, quiz 714-715; and Lopez-Garcia et al., Am J Clin Nutr., 2006, 84:888-893), in diabetics (Lopez-Garcia et al., id.) and in the obeses (Arsenault et al., Eur J Clin Nutr., 2009; 63:1419-1424).

US patent application 2010/0112098 discloses the inhibiting effect of an extract of green coffee obtained from *Coffea robusta* on the glucose-6-phosphatase enzyme and the use of such extract to reduce glycemia, and consequently body weight and the risk of onset of type 2 diabetes, in subjects. According to this document, the inhibition of glucose-6-phosphatase which underlies the effects attributed to the extract of green coffee is due to what are known as chlorogenic acids, a family of esters of certain hydroxycinnamic acids (caffeic acid, ferulic acid and p-coumaric acid) with (-)-quinic acid, which are naturally present in green coffee. Since during the roasting process a part of the natural content of chlorogenic acids of green coffee is destroyed, according to US 2010/0112098 it is thus advisable to avoid roasting coffee beans, in order to preserve their high natural content of chlorogenic acids, and use only an extract of green coffee (i.e. not roasted).

WO 2011/134656 discloses a method for preparing a blend of roasted coffee from which in turn a beverage is prepared which has a high content of chlorogenic acids and of N-methylpyridine cations, a reduced content of carboxylic acid-5-hydroxytryptamides and which is attributed a higher antioxidant capacity. According to the above document, the specific concentrations of the above mentioned compounds are responsible for the physiological properties of the coffee blend described therein. In order to obtain a product that has such concentrations and at the same time adequate sensorial features, the method described necessitates the blending of two types of roasted coffee, each obtained by way of a different roasting. Furthermore, even though the specific composition of bioactive compounds of the blend is attributed antioxidant properties, WO 2011/134656 does not describe the existence of effects on the metabolism of glucose by the blend, nor does it mention a preventive effect against type 2 diabetes.

The aim of the present invention is thus to provide a roasted coffee characterized by a specific composition of bioactive compounds which is useful for the prevention of type 2 diabetes.

Within this aim, an object of the invention is to provide a roasted coffee that has anti-inflammatory and antioxidant properties and which is at the same time effective in favoring the metabolism of glucose.

Another object of the invention is to provide a product that is useful for preventing type 2 diabetes, which has a low cost and which prevention can be practiced with ease and convenience by the consumer.

Another object of the present invention is to provide a method for preparing a roasted coffee having a specific composition of bioactive compounds which is useful for preventing type 2 diabetes, and at the same time suitable for preparing beverages that have organoleptic characteristics that can be appreciated by the consumer.

Another object of the invention is to provide a method for preparing a roasted coffee as described herein which is highly reliable, easy to implement and having low cost.

According to a first aspect of the invention, this aim and these and other objects which will become better apparent hereinafter are achieved by a roasted coffee of *Coffea arabica* var. *Laurina*, characterized in that a powder of said coffee comprises 5.0 to 10.0 mg/g trigonelline and 3.0 to 8.0 mg/g mono-caffeoylquinic acids.

According to another aspect of the invention, the set aim and objects are achieved by a coffee beverage prepared with a roasted coffee as described herein.

According to another aspect of the invention, the set aim and objects are achieved by a capsule for extracting a beverage from a powdered substance by way of water under pressure, wherein the powdered substance is a powder of the roasted coffee as described herein.

According to another aspect of the invention, the set aim and objects are achieved by a method for the preparation of a roasted coffee as described herein, characterized in that said method comprises the step of roasting beans of *Coffea arabica* var. *Laurina* at a temperature and for a time which are such as to achieve an average decrease of the total weight of the coffee beans greater than or equal to 16.6 ± 1.2%.

Finally, according to another aspect of the invention, the set aim and objects are achieved by a roasted coffee as described herein for use in the prevention of type 2 diabetes.

Further characteristics and advantages of the invention will become better apparent from the detailed description that follows and from the accompanying drawings wherein:

Figure 1 is a chart of the plasmatic concentrations of C reactive protein (CRP) before (washout) and after the consumption of coffee beverages of products A (comparative), B and C (according to the invention);

Figure 2 is a chart of the erythrocyte concentrations of glutathione before (washout) and after the consumption of coffee beverages of products A (comparative), B and C (according to the invention);

Figure 3 is a chart of the HOMA index of insulin resistance before (washout) and after the consumption of coffee beverages of products A (comparative), B and C (according to the invention);

Figure 4 is a chart of the insulinogenic index before (washout) and after the consumption of coffee beverages of products A (comparative), B and C (according to the invention).

In the following description of the invention and in the appended claims, the terms listed below have the meaning given in the respective definitions.

The term "mono-caffeoylquinic acids" refers to the group of monoesters of caffeic acid with (-)-quinic acid consisting of 3-caffeoylquinic acid, 4-caffeoylquinic acid, and 5-caffeoylquinic acid.

The term "mono-feruloylquinic acids" refers to the group of monoesters of ferulic acid with (-)-quinic acid consisting of 3-feruloylquinic acid, 4-feruloylquinic acid, and 5-feruloylquinic acid.

The term "dicaffeoylquinic acids" refers to the group of diesters of caffeic acid with (-)-quinic acid consisting of 3,4-dicaffeoylquinic acid, 3,5-dicaffeoylquinic acid, and 4,5-dicaffeoylquinic acid.

As discussed in more detail below, the roasted coffee according to the present invention is characterized by a specific composition of bioactive compounds which is such as to render this product particularly useful for the preventive treatment of type 2 diabetes.

The inventors of the present invention have unexpectedly discovered that the above mentioned roasted coffee can be prepared by way of a method described herein from beans of *Coffea arabica* variety (var.) *Laurina.* The *Laurina* variety is a natural mutant of *Coffea arabica Bourbon,* which was first discovered on the island of Reunion. Such variety is now also known by the name "Bourbon Low Caffeine" (BLC).

The peculiar characteristic of the *Laurina* variety is a reduced caffeine content compared to other varieties of *Coffea arabica,* to which must be added sensory characteristics, which emerged from studies conducted by the applicant, which make the *Laurina* variety ideal for being subjected to more intense roasting processes.

According to the first aspect of the invention, the roasted coffee of *Coffea arabica* var. *Laurina* of the present invention is characterized in that a powder thereof, for example obtained by grinding, comprises the following compounds in the respective quantities expressed in mg per gram of powder (mg/g):

- trigonelline                      5.0 - 10.0 mg/g; and
- mono-caffeoylquinic acids     3.0 - 8.0 mg/g.

The roasted coffee of the present invention can further contain a total quantity of oil of up to 18% by weight of the powder, preferably a quantity comprised between 14% and 17.5%.

Preferably, the roasted coffee of the present invention has an average color lower than $91 \pm 3$ AU (Arbitrary Units) as measured on a powder of such coffee by means of a Colorette 3B colorimeter manufactured by Probat.

The measurement of the average color of the roasted coffee is carried out on the roasted coffee, suitably ground and according to the protocols provided by the maker of the colorimeter, setting the internal reference of the device to 200 AU.

In a preferred embodiment of the roasted coffee of the present invention, the powder of the roasted coffee comprises:

|  |  |
|---|---|
| - trigonelline | 5.0 - 6.5 mg/g; and |
| - mono-caffeoylquinic acids | 3.0 - 4.5 mg/g. |

In this preferred embodiment, the roasted coffee of the present invention can further contain a total quantity of oil equal to $15 \pm 1\%$ by weight of the powder.

According to the preferred embodiment, the roasted coffee can have an average color lower than or equal to $62 \pm 3$ AU as measured on a powder of such coffee by means of a Colorette 3B colorimeter manufactured by Probat.

Also, in this preferred embodiment, the roasted coffee of the present invention can be characterized in that a beverage prepared with such roasted coffee comprises the following compounds in the respective quantities expressed in mg per liter of beverage (mg/L):

|  |  |
|---|---|
| - caffeine | $1850 \pm 10\%$ mg/L; |
| - trigonelline | $1150 + 10\%$ mg/L; |
| - N-methylpyridine | $470 \pm 10\%$ mg/L; |
| - niacin | $105 \pm 10\%$ mg/L; |
| - mono-caffeoylquinic acids | $1300 \pm 10\%$ mg/L; |
| - mono-feruloylquinic acids | $185 \pm 15\%$ mg/L; |
| - dicaffeoylquinic acids | $16 \pm 15\%$ mg/L; |
| - caffeic acid | $7.5 + 15\%$ mg/L; and |
| - ferulic acid | $6.6 \pm 15\%$ mg/L. |

Preferably, a roasted coffee according to the above preferred embodiment is the "Product C" of Example 1 which has the characteristics given in Table V and a beverage prepared with such product has the characteristics shown in Table VI.

In a particularly preferred embodiment of the roasted coffee of the present invention, the powder of the roasted coffee comprises:

|  |  |
|---|---|
| - trigonelline | 8.5 - 10.0 mg/g; and |
| - mono-caffeoylquinic acids | 6.5 - 8.0 mg/g. |

In this particularly preferred embodiment, the roasted coffee of the present invention can further contain a total quantity of oil equal to $16.5 \pm 1\%$ by weight of the powder.

According to the particularly preferred embodiment, the roasted coffee can have an average color comprised between $62 \pm 3$ AU and $82 \pm 3$ AU as measured on a powder of such coffee by means of a Colorette 3B colorimeter manufactured by Probat.

Also, in this particularly preferred embodiment, the roasted coffee of the present invention can be characterized in that a beverage prepared with such roasted coffee comprises the following compounds in the respective quantities expressed in mg per liter of beverage (mg/L):

|  |  |
|---|---|
| - caffeine | $1900 \pm 10\%$ mg/L; |
| - trigonelline | $1850 \pm 10\%$ mg/L; |
| - N-methylpyridine | $340 \pm 10\%$ mg/L; |
| - niacin | $105 \pm 10\%$ mg/L; |
| - mono-caffeoylquinic acids | $2150 \pm 10\%$ mg/L; |
| - mono-feruloylquinic acids | $290 \pm 15\%$ mg/L; |

(continued)

| | |
|---|---|
| - dicaffeoylquinic acids | 27 ± 15% mg/L; |
| - caffeic acid | 10 + 15% mg/L; and |
| - ferulic acid | 3.8 ± 15% mg/L. |

Preferably, a roasted coffee according to the above particularly preferred embodiment is the "Product B" of Example 1 which has the characteristics given in Table III and a beverage prepared with such product has the characteristics shown in Table IV.

Obviously, the roasted coffee of the present invention according to all the above embodiments can be in the form of beans or in the form of powder, the latter obtained for example by way of conventional grinding methods known to the person skilled in the art.

The roasted coffee of the present invention can be used for preparing coffee beverages according to conventional methods, for example by way of infusion in cold or hot water or infusion by way of a coffee machine at atmospheric pressure or under pressure.

According to another aspect of the invention, a coffee beverage prepared with the roasted coffee of *Coffea arabica* var. *Laurina* described herein constitutes a further object of the invention.

In a preferred embodiment, the coffee beverage of the present invention comprises the following compounds in the respective quantities expressed in mg per liter of beverage (mg/L):

| | |
|---|---|
| - caffeine | 1850 ± 10% mg/L; |
| - trigonelline | 1150 ± 10% mg/L; |
| - N-methylpyridine | 470 ± 10% mg/L; |
| - niacin | 105 ± 10% mg/L; |
| - mono-caffeoylquinic acids | 1300 ± 10% mg/L; |
| - mono-feruloylquinic acids | 185 ± 15% mg/L; |
| - dicaffeoylquinic acids | 16 ± 15% mg/L; |
| - caffeic acid | 7.5 + 15% mg/L; and |
| - ferulic acid | 6.6 ± 15% mg/L. |

In a particularly preferred embodiment, the coffee beverage of the present invention comprises the following compounds in the respective quantities expressed in mg per liter of beverage (mg/L):

| | |
|---|---|
| - caffeine | 1900 ± 10% mg/L; |
| - trigonelline | 1850 ± 10% mg/L; |
| - N-methylpyridine | 340 ± 10% mg/L; |
| - niacin | 105 ± 10% mg/L; |
| - mono-caffeoylquinic acids | 2150 ± 10% mg/L; |
| - mono-feruloylquinic acids | 290 ± 15% mg/L; |
| - dicaffeoylquinic acids | 27 ± 15% mg/L; |
| - caffeic acid | 10 + 15% mg/L; and |
| - ferulic acid | 3.8 ± 15% mg/L. |

According to another aspect of the invention, the present invention also relates to a capsule for the extraction of a beverage from a powdered substance by means of pressurized water, wherein the powdered substance is a powder of the roasted coffee of the present invention.

Preferably, the above mentioned capsule comprises a boxlike body containing the powdered substance, the boxlike body having a base provided with an output port for the outflow of the extracted beverage, where the outflow port can be optionally closed by a crema forming element or by a weakened region that can be opened by pressurized water.

Preferred embodiments of the above mentioned capsule are disclosed by way of non-limiting example in WO 2008/011913 and in Italian patent application MI2013A000906, both in the name of Illycaffé SpA. An example of such capsule is represented by the capsules of the system known as "Iperespresso®" marketed by Illycaffé SpA.

The roasted coffee of the present invention is prepared according to a method that involves roasting green coffee beans of *Coffea arabica* var. *Laurina* at a roasting temperature and for a roasting time which are such as to obtain a roasted coffee that has the content di bioactive compounds as described herein.

In particular, according to another aspect of the invention, the method for preparing a roasted coffee according to the invention is characterized in that it comprises the step of roasting green coffee beans of *Coffea arabica* var. *Laurina* at a roasting temperature and for a time so as to achieve an average decrease of the total weight of the coffee beans greater than 16.6 + 1.2%.

It should be noted that the above mentioned average decrease of the total weight corresponds to an average color value of the roasted coffee lower than 91 ± 3 AU, as measured with a Colorette 3B colorimeter manufactured by Probat on the conveniently ground roasted coffee.

For the preparation of a roasted coffee according to the preferred embodiment described previously, the method of the invention involves that the step of roasting be carried out at a temperature and for a time so as to achieve an average decrease of the total weight of the coffee beans greater than or equal to 18.5 ± 1.2%. This average decrease of the weight corresponds to an average color value of the roasted coffee lower than or equal to 62 ± 3 AU, as measured on a powder of the roasted coffee by means of a Colorette 3B colorimeter manufactured by Probat.

For the preparation of a roasted coffee according to the particularly preferred embodiment described previously, the method of the invention involves that the step of roasting be carried out at a temperature and for a time so as to achieve an average decrease of the total weight of the coffee beans greater than 16.6 ± 1.2% and lower than 18.5 ± 1.2%. This average decrease of the weight corresponds to an average color value of the roasted coffee comprised between 62 + 3 AU and 82 ± 3 AU, as measured on a powder of such coffee by means of a Colorette 3B colorimeter manufactured by Probat.

As already explained previously, the determination of the color of the roasted coffee with the Probat Colorette 3B colorimeter is carried out according to the protocols provided by the maker of the device, setting the internal reference of the colorimeter to 200 AU.

By way of the method of the present invention, the green coffee beans are subjected to a more intense roasting than that adopted for the preparation of conventional roasted coffees. Usually, with such an intense roasting, products are obtained which have sensory characteristics that are inadequate for use in the preparation of beverages with organoleptic characteristics that are acceptable to the consumer. However, the roasted coffee that can be obtained with the method of the present invention presents sensory characteristics that do not compromise the acceptability of the beverages by the consumer.

The roasting step of the method of the present invention is not limited to the use of particular roasting devices, and substantially any roasting device can be used which is known to the person skilled in the art. By way of non-limiting example, the roasting step can be carried out by means of using a drum roasting device or by means of using a fluid bed rotating roasting device.

The roasted coffee obtainable by the method of the present invention also constitutes an aspect of the present invention. Although not explicitly repeated here, the embodiments of the roasted coffee described previously are to be considered valid also with reference to the roasted coffee obtainable by the method of the present invention.

According to a further aspect of the invention, the present invention also relates to the roasted coffee of the present invention for use in the prevention of type 2 diabetes.

The inventors of the present invention have unexpectedly observed that consumption of the roasted coffee of the present invention, in particular in the form of a beverage, has metabolic protection effects that favor the prevention of type 2 diabetes, such as an improvement of the systemic inflammatory state, an increase in antioxidant protection capacity, and an improvement of insulin secretion stimulus and of insulin sensitivity. Without wishing to be bound to any particular theory, it is believed that the above mentioned metabolic protection effects are caused by the peculiar composition of bioactive compounds found in the roasted coffee of the present invention.

In a preferred embodiment of the roasted coffee for use in the prevention of type 2 diabetes, the coffee is consumed in the form of a beverage. For example, the beverage can be prepared with a capsule for the extraction of a beverage from a powdered substance as described previously.

Preferably, for the purposes of preventing type 2 diabetes, consumption of the beverage is daily, for example up to 4 times a day. Preferably, the beverage is consumed 4 times a day.

Although the roasted coffee according to the invention has been described in particular for use in the prevention of type 2 diabetes, it can also be used for the prevention of metabolic alterations associated with diabetes, such as for example dyslipidemia, activation of low-grade systemic inflammation, and hypertension.

Finally, in a further aspect of the invention, the present invention also relates to a method for the prevention of type 2 diabetes, which is characterized in that such method comprises the step of administering the roasted coffee of the present invention to a subject.

It should be understood that the characteristics of the embodiments described with reference to the roasted coffee for use in the prevention of type 2 diabetes are to be considered valid also with reference to the above mentioned method for the prevention of type 2 diabetes, even if they are not explicitly repeated.

The invention will now be described with reference to the following non-limiting examples.

EXAMPLE 1

Three samples of roasted coffee were prepared, identified below as "product A", "product B" and "product C", using two commercial batches of green coffee of *Coffea arabica* var. *Laurina.*

The green coffee was roasted on a pilot scale at different degrees of roasting, ground and packaged in capsules to be used for the preparation of the beverages consumed over the course of the clinical trial described below in Example 3.

Each of products A, B and C was chemically characterized both in powder form and in beverage form, the latter prepared by extraction from an Iperespresso® capsule by way of pressurized water using a coffee machine compatible with the Iperespresso® system.

The data corresponding to such characterization are given in the following Tables I-VI, in which the quantities shown of mono-caffeoylquinic acids correspond to the sum of the quantities of 3-mono-caffeoylquinic acid, 4-mono-caffeoylquinic acid and 5-mono-caffeoylquinic acid; the quantities shown of mono-feruloylquinic acids correspond to the sum of the quantities of 3-feruloylquinic acid, 4-feruloylquinic acid, and 5-feruloylquinic acid; and the quantities shown of dicaffeoylquinic acids correspond to the sum of the quantities of 3,4-dicaffeoylquinic acid, 3,5-dicaffeoylquinic acid, and 4,5-dicaffeoylquinic acid.

The average color of products A, B and C was measured on the roasted coffee, suitable ground, by means of a Colorette 3B colorimeter manufactured by Probat, according to the protocols provided by the maker and setting the internal reference of the colorimeter to 200 AU.

PRODUCT A (comparative)

Product A was prepared by carrying out a series of roastings in a pilot plant with the following roasting conditions:

- quantity of coffee per batch: 8 kg;
- temperature of the burner: 410°C;
- final temperature: 185-186°C;
- roasting time: 11 minutes;
- average color: 108 ± 3 AU.

The product in beans was ground according to standard methods and the powder thus obtained was packaged in Iperespresso® capsules.

The tables below show the data for the chemical characterization of the powder of product A (Table I) and of the corresponding beverage (Table II).

Table I

| Compound | Quantity in powder A |
|---|---|
| Trigonelline | 12.5 ±0.5 mg/g |
| Mono-caffeoylquinic acids | 15.8 ±2.0 mg/g |
| Total oil | 14.4 ± 1% |

For the beverage, below are the quantities of the individual compounds in mg for an average volume of 28.8 ± 1.9 mL. The margin for error is comprised between 5 and 10%.

Table II

| Compound | Quantity in the beverage |
|---|---|
| Caffeine | 49.3 mg |
| Trigonelline | 83.5 mg |
| N-methylpyridine | 4.3 mg |
| Niacin | 2.5 mg |
| Mono-caffeoylquinic acids | 107.5 mg |
| Mono-feruloylquinic acids | 10.6 mg |
| Dicaffeoylquinic acids | 2.8 mg |

(continued)

| Compound | Quantity in the beverage |
| --- | --- |
| Caffeic acid | 0.4 mg |
| Ferulic acid | 0.1 mg |
| Total oil | 86.8 mg |

PRODUCT B (invention)

Product B was prepared by carrying out a series of roastings in a pilot plant with the following roasting conditions:

- quantity of coffee per batch: 8 kg;

- temperature of the burner: 400°C;

- final temperature: 197°C;

- roasting time: 13 minutes;

- average color: 82 ± 3 AU.

The product in beans was ground according to standard methods and the powder thus obtained was packaged in Iperespresso® capsules.

The tables below show the data for the chemical characterization of the powder of product B (Table III) and of the corresponding beverage (Table IV).

Table III

| Compound | Quantity in powder B |
| --- | --- |
| Trigonelline | 9.2 ±0.5 mg/g |
| Mono-caffeoylquinic acids | 7.3 ±0.6 mg/g |
| Total oil | 16.5 ± 1% |

For the beverage, below are the quantities of the individual compounds in mg for an average volume of 25.7 ± 2.2 mL. The margin for error is comprised between 5 and 10%.

Table IV

| Compound | Quantity in the beverage |
| --- | --- |
| Caffeine | 49.2 mg |
| Trigonelline | 47.8 mg |
| N-methylpyridine | 8.8 mg |
| Niacin | 2.72 mg |
| Mono-caffeoylquinic acids | 56.4 mg |
| Mono-feruloylquinic acids | 7.5 mg |
| Dicaffeoylquinic acids | 0.7 mg |
| Caffeic acid | 0.3 mg |
| Ferulic acid | 0.1 mg |
| Total oil | 56.6 mg |

PRODUCT C (Invention)

Product C was prepared by carrying out a series of roastings in a pilot plant with the following roasting conditions:

- quantity of coffee per batch: 8 kg;

- temperature of the burner: 390°C;

- final temperature: 201-202°C;

- roasting time: 14 minutes;

- average color: 62 ± 3 AU.

The product in beans was ground according to standard methods and the powder thus obtained was packaged in Iperespresso® capsules.

The tables below show the data for the chemical characterization of the powder of product C (Table V) and of the corresponding beverage (Table VI).

Table V

| Compound | Quantity in powder C |
|---|---|
| Trigonelline | 5.7 ±0.3 mg/g |
| Mono-caffeoylquinic acids | 3.8 ±0.3 mg/g |
| Total oil | 15.2 ± 1% |

For the beverage, below are the quantities of the individual compounds in mg for an average volume of 23.9 ± 2.2 mL. The margin for error is comprised between 5 and 10%.

Table VI

| Compound | Quantity in the beverage |
|---|---|
| Caffeine | 44.7 mg |
| Trigonelline | 28.1 mg |
| N-methylpyridine | 11.3 mg |
| Niacin | 2.5 mg |
| Mono-caffeoylquinic acids | 31.4 mg |
| Mono-feruloylquinic acids | 4.5 mg |
| Dicaffeoylquinic acids | 0.4 mg |
| Caffeic acid | 0.2 mg |
| Ferulic acid | 0.2 mg |
| Total oil | 71.5 mg |

It should be noted that the method of roasting used here for the preparation of product A (comparative) is less intense than the conventional roasting methods that are commonly used with varieties of coffee other than *Laurina*. The roasting methods used for products B and C (according to the invention) are progressively increasingly more intense than the conventional methods.

EXAMPLE 2

A sensory characterization was carried out on products A, B and C prepared in Example 1, by submitting the beverages obtained using the capsules of products A, B and C prepared in Example 1 for examination by a panel of eight expert judges in sensory characterization.

The products were characterized in two sessions, with tests conducted in duplicate in order to obtain information on the degree of agreement between the participants in the tests. In the two sessions, the judges gave marks of between 0 and 7 to the sensory attributes used to characterize the products.

The sensory analysis was carried out in an accredited laboratory (ISO 17025:2005), equipped according to the ISO 8589 standard. The acquisition software used is the Fizz Network software (version 2.31G, Biosystemes, Couternon, France) while the data were processed using the XLSTAT 2006.4 and Fizz programs.

The analysis of variance (ANOVA) shows that almost all the attributes used for the characterization of the products are useful in that they are statistically different, with the exception of caramel.

Table VII for the ANOVA shows the average values of the marks assigned by the judges to the various attributes used for the characterization of the products A, B and C and the p-value.

As can be seen from an examination of the data in Table VII, all the sensory attributes evaluated can be discriminated with the exception of caramel. It is further noted that there is a different acid/bitter balance between the products examined: product B is substantially balanced, while product A is skewed toward acid and product C toward bitter. All the beverages in any case have organoleptic characteristics that are acceptable to the consumer.

Table VII

| Attribute | A | B | C | p-value |
|---|---|---|---|---|
| Bitter | 4.5 | 5.5 | 6.8 | < 0.0001 |
| Acid | 5.6 | 4.6 | 3.4 | < 0.0001 |
| Sweet | 4.7 | 4.3 | 2.5 | < 0.0001 |
| Body | 4.7 | 5.6 | 5.6 | 0.000 |
| Overall aroma | 5.5 | 6.1 | 6.6 | 0.000 |
| Chocolate | 2.8 | 4.1 | 3.1 | 0.009 |
| Caramel | 2.4 | 3.1 | 2.3 | 0.187 |
| Toasted bread | 1.8 | 3.0 | 3.1 | 0.006 |
| Flower/Fruit | 2.2 | 1.7 | 1.5 | 0.082 |
| Burned | 1.3 | 2.4 | 4.4 | < 0.0001 |
| Overall quality | 4.5 | 5.9 | 3.2 | < 0.0001 |

The sensory analysis thus fully confirms that a method of intense roasting according to the present invention does not compromise the acceptability of the beverages prepared with the roasted coffee that can be obtained with such method.

EXAMPLE 3

A clinical trial was carried out on healthy volunteers in order to evaluate the effects of beverages prepared with the roasted coffee of products A, B and C of Example 1 on the metabolism of glucose (focusing on insulin sensitivity) and on the mechanisms associated with an altered metabolism of glucose (in particular low-grade systemic inflammation and oxidative stress).

Trial protocol

The trial was conducted in two trial campaigns on 14 healthy male subjects who signed the informed-consent release form for the trial. In the first campaign, products A and B were tested, while in the second campaign product C was tested.

For the duration of the trial, the participating subjects were asked to monitor their dietary intake, in order to standardize the intake of polyphenolic compounds. In particular, the subjects were asked to: maintain the dietary habits of the last six months, unaltered; peel fruit and potatoes (the concentration of polyphenols is higher in peel and in the outer parts of fruit and vegetables); avoid old potatoes or potatoes that showed variations of color after cooking or conservation; avoid introducing juices (whether fresh or from concentrate or smoothies) of fruit and vegetables into their diet, and to choose fresh produce; avoid smoked products (fish, cold meats etc.), herbalists' products, and food supplements.

Furthermore, in order to control the effects of alcohol and physical activity on the glucose metabolism, for the duration of the trial subjects were asked to maintain their normal levels of physical activity and to maintain their normal consumption of alcohol if less than or equal to 2 alcoholic beverages/day (one alcoholic beverage = 120 ml of average-strength wine; 330 ml of beer, or one can; 30-40 ml of spirits) or to reduce consumption to no more than 2 alcoholic beverages/day, if their habitual intake is higher.

The subjects were studied in 3 different phases (two of which were conducted during the first campaign and one during the second campaign). All 14 subjects completed the first campaign, while two of the 14 subjects had to withdraw from the second campaign, for personal reasons not linked to the trial.

Each phase was organized as follows:

- 1st week: "washout" period. In this week the subjects were asked to abstain from consumption of any beverage and food containing coffee or caffeine (tea, energy drinks, hot chocolate, chocolate etc.);
- 2nd week: in the second week the subjects were asked to consume 4 coffee beverages per day (product A, B or C as indicated below), to be consumed in the morning at breakfast, at mid-morning, after lunch and in the late afternoon (from 4pm onward), while continuing to abstain from taking any other stimulating substance as in the washout period.

During the two campaigns, the subjects prepared a beverage with the roasted coffee of product A (campaign 1, phase 1), B (campaign 1, phase 2) or C (campaign 2, phase 1) using the capsules prepared in Example 1. In order to standardize the method of preparation of the beverage, all the subjects were issued with an identical model of a coffee machine compatible with the Iperespresso® system (Francis Francis X7 model, sold by Illycaffé S.p.A.) and with special beakers. The subjects were instructed in the preparation of the beverage with the above mentioned coffee machine and were instructed to fill the beakers with the beverage with a volume corresponding to the upper part of a logo which was reproduced identically on each beaker.

At the end of each campaign, the subjects were subjected to the analyses described below in order to evaluate the effects of the consumed beverage on insulin sensitivity, on the degree of systemic inflammation and on oxidative stress.

Oral Glucose Tolerance Test (OGTT)

The OGTT, or the glucose load curve, is a standard method, used in clinical medicine, for determining insulin sensitivity. The test was conducted on the subjects after fasting, at about 8.00-8.30 in the morning. In particular, each subject received 75 g of glucose dissolved in a beaker of water (300 ml). By way of a venous access prepared in advance by a specialist nurse, 9 blood samples were taken, exactly 30 and 15 minutes before the glucose loading, immediately before the glucose loading ($T_0$), and at 30, 60, 90, 120, 150 and 180 minutes after the glucose loading. At each sample 3 mL of blood was taken, except for the sample taken immediately before the glucose loading (9 mL), for a total of 33 mL of blood taken for each subject.

The blood samples were immediately subjected to centrifuge at 3000 g for 10 minutes at 4°C. Plasma and red blood cells were then suitably aliquoted and stored at -20°C.

The evaluation of insulin sensitivity was carried out by way of determination of the insulin and glycemia plasma levels in the samples taken at each time point.

The data obtained were then used to calculate the principal indices of insulin resistance (HOMA index and the area under the curve for insulin level and glycemia level as a function of time) and the insulinogenic index.

The HOMA index was calculated using the following formula:

$$HOMA = \frac{\left( glycemia\ level\ after\ fasting\ X\ insulin\ level\ after\ fasting \right)}{405}$$

where the glycemia level is expressed in mg/dL and the insulin level is expressed in $\mu$mol/mL.

Statistical analysis of the data for the HOMA index was carried out by way of analysis of covariance (ANCOVA) for repeated measurements using the average values for washout as the covariate, in order to determine the effect of the period (washout vs. consumption of coffee) and of the product (A, B and C).

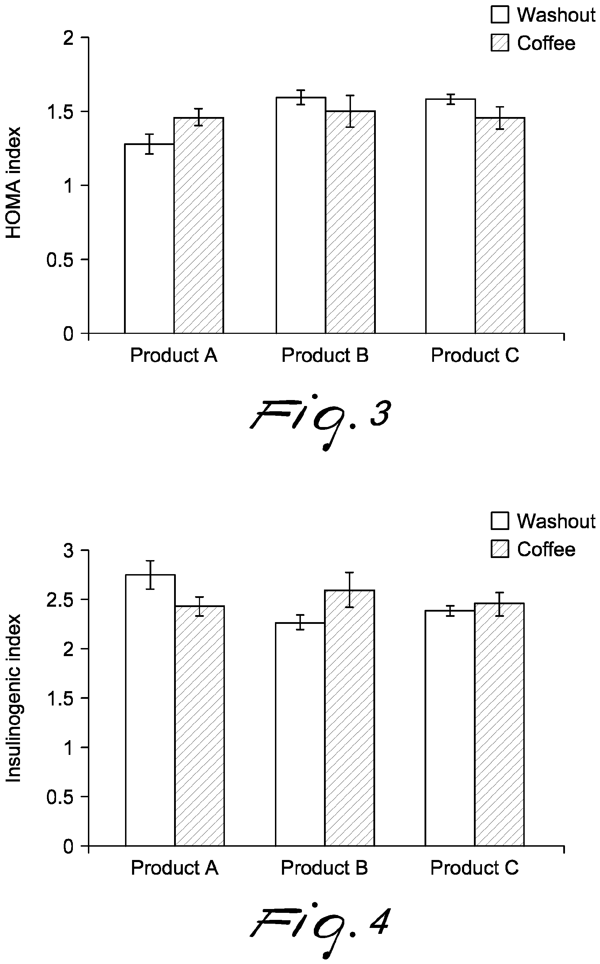

Figure 3 shows a chart of the values of the HOMA index before (washout) and after the consumption of coffee beverages of products A, B and C.

In order to calculate the area under the curve (AUC) of insulin and glycemia levels, the values of the concentrations of insulin and glycemia obtained from the blood samples were plotted on a chart as a function of time, and the AUC of the curve thus obtained was then calculated with the trapezoidal method.

The insulinogenic index was calculated as the product of the Matsuda index and the ratio between the AUC of insulin levels and the AUC of glycemia levels. The Matsuda Index, in turn, was calculated as follows :

$$\text{Matsuda Index} = 10000 \, / \, \sqrt{\left( glyc_{T0} * ins_{T0} * ins_{avgOGTT} * glyc_{avg}OGTT \right)}$$

where $glyc_{T0}$ is the basal glycemia level after fasting, $ins_{T0}$ is the basal insulin level after fasting, $ins_{avgOGTT}$ is the average of the insulin levels measured during OGTT and $glyc_{avgOGTT}$ is the average of the glycemia levels measured during OGTT.

Statistical analysis of the data for the insulinogenic index was carried out by way of ANCOVA for repeated measurements using the average values for washout as the covariate, in order to determine the effect of the period (washout vs. consumption of coffee) and of the product (A, B and C).

Figure 4 shows a chart of the values of the insulinogenic index before (washout) and after the consumption of coffee beverages of products A, B and C.

Other analyses carried out

Using the same blood samples taken from the subjects for the OGTT, further analyses were carried out aimed at determining the plasma concentration values of C reactive protein (CRP) and of the erythrocyte glutathione concentration.

Evaluation of the plasma concentration of CRP was carried out at a certified laboratory, by way of standard immunoturbidimetric analyses. Statistical analysis of the data for the plasma concentration of CRP was carried out by way of ANCOVA for repeated measurements using the average values for washout as the covariate, in order to determine the effect of the period (washout vs. consumption of coffee) and of the product (A, B and C).

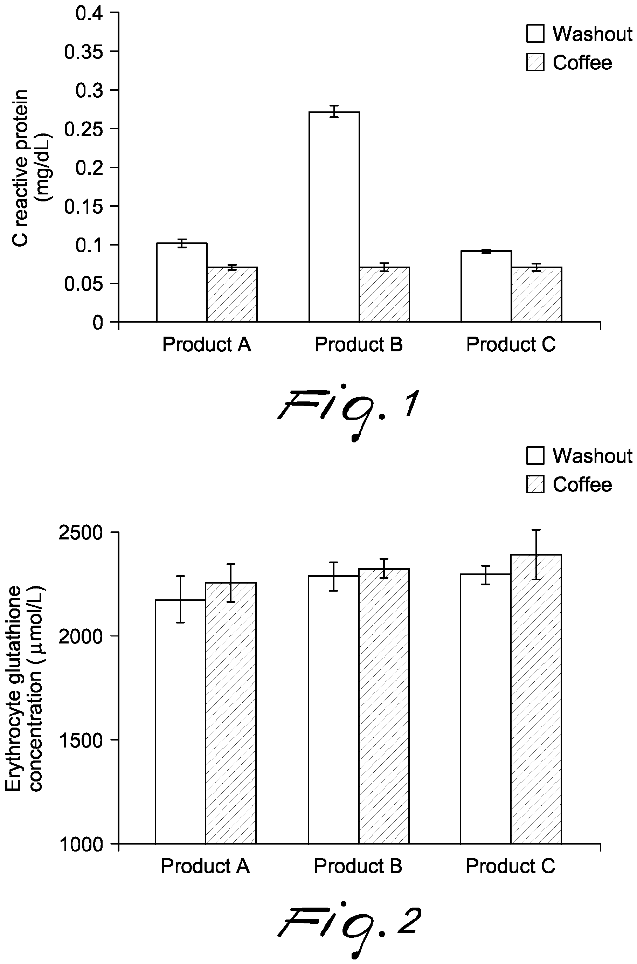

Figure 1 shows a chart of the values of the plasma concentration of CRP before (washout) and after the consumption of coffee beverages of products A, B and C.

Evaluation of the erythrocyte glutathione concentration was carried out by way of gas chromatography/mass spectrometry (GC/MS) while monitoring the mass/load ratio (m/z-1) 363-366 and using the internal standard method, by way of the addition to the sample to be analyzed of known quantities of $[^{13}C_2, {}^{15}N\text{-glycine}]$glutathione. Statistical analysis of the data for the erythrocyte glutathione concentration was carried out by way of ANCOVA for repeated measurements using the average values for washout as the covariate, in order to determine the effect of the period (washout vs. consumption of coffee) and of the product (A, B and C).

Figure 2 shows a chart of the values of the erythrocyte glutathione concentration before (washout) and after the consumption of coffee beverages of products A, B and C.

Results and conclusions

Despite the consumption in moderate doses (4 espresso coffee servings per day), and independently of the specific product consumed, coffee induces an improvement of the inflammatory state with decrease of the CRP inflammation marker (see the data in Figure 1), as well as an improvement of the total antioxidant capacity, with a significant increase in the concentration of intracellular glutathione, the main antioxidant of the human body (see the data in Figure 2).

The activation of the inflammatory state and the redox unbalancing which promotes the increase of oxidative stress are two mechanisms associated with the development of various metabolic alterations, including the development of type 2 diabetes. The foregoing results show therefore that the roasted coffee according to the invention is capable of combating such mechanisms and favoring the prevention of type 2 diabetes.

Unexpectedly, we have further observed specific metabolic effects on glucose metabolism and insulin sensitivity depending on the type of coffee consumed. As shown in Figure 3, the HOMA index increases with product A (comparative) while it improves significantly with products B and C (invention), thus showing a protective effect against the development of diabetes by the roasted coffee of the present invention, of which products B and C are examples.

Similar results are obtained for the insulinogenic index measured during the oral loading of glucose. The insulinogenic index describes the capacity of the pancreatic β cells to secrete insulin in response to the increase of blood concentrations of glucose: the greater the response of the β cells, the greater the efficiency with which the glucose is stored by the body, and the lower the risk of developing type 2 diabetes. As shown in Figure 4, the insulinogenic index is also increased in response to consumption of products B and C according to the invention, while it worsens in response to the comparative product A.

In view of the efficacy in reducing insulin resistance and improving insulinogenic capacity, product C and especially product B according to the invention thus have characteristics that are suitable for the prevention of the development of type 2 diabetes.

In practice it has been found that the roasted coffee according to the invention fully achieves the set aim, in that its composition of bioactive compounds is characterized by anti-inflammatory and antioxidant properties, it reduces insulin resistance and it improves insulinogenic capacity, and thus is ideal for the preventive treatment of type 2 diabetes.

Moreover, the roasted coffee according to the invention can be used to carry out the prevention of type 2 diabetes in a practical and simple manner and at low cost, as such preventive use is based simply on the daily consumption of coffee, a habit that is already widespread among consumers.

Finally, the method for preparing roasted coffee according to the invention on the one hand makes it possible to obtain a roasted coffee that has a composition of bioactive compounds which is useful for the prevention of type 2 diabetes and with which it is possible to prepare beverages that have appreciable organoleptic characteristics, thanks to the use of a specific raw material and of a more intense roasting than the conventional methods, and on the other hand it is simple to carry out and has low cost, in that it does not require the use of specially-designed devices, it being possible to use conventional roasting devices.

**Claims**

1. A roasted coffee of *Coffea arabica* var. *Laurina*, **characterized in that** a powder of said coffee comprises:

   - 5.0 to 10.0 mg/g trigonelline; and
   - 3.0 to 8.0 mg/g mono-caffeoylquinic acids.

2. The roasted coffee according to claim 1, wherein the powder of said roasted coffee has an average color lower than 91 ± 3 AU (Arbitrary Units), measured by means of a Colorette 3B colorimeter manufactured by Probat.

3. The roasted coffee according to claim 1, wherein the powder comprises:

   - 8.5 to 10.0 mg/g trigonelline; and
   - 6.5 to 8.0 mg/g mono-caffeoylquinic acids.

4. The roasted coffee according to claim 3, wherein the powder of said roasted coffee has an average color comprised between 62 ± 3 AU and 82 ± 3 AU, measured by means of a Colorette 3B colorimeter manufactured by Probat.

5. The roasted coffee according to claim 1, wherein the powder comprises:

   - 5.0 to 6.5 mg/g trigonelline; and
   - 3.0 to 4.5 mg/g mono-caffeoylquinic acids.

6. The roasted coffee according to claim 5, wherein the powder of said roasted coffee has an average color lower than or equal to 62 ± 3 AU, measured by means of a Colorette 3B colorimeter manufactured by Probat.

7. A coffee beverage prepared with a roasted coffee according to any of the preceding claims.

8. A capsule for extracting a beverage from a powdered substance by means of pressurized water, said capsule **characterized in that** the powdered substance is a powder of roasted coffee according to any of claims 1-6.

9. A method for preparing a roasted coffee according to any of claims 1-6, **characterized in that** said method comprises the step of roasting green coffee beans of *Coffea arabica* var. *Laurina* at a temperature and for a time so as to achieve an average decrease of the total weight of the coffee beans greater than 16.6 ± 1.2%.

10. The method according to claim 9 for preparing a roasted coffee according to claim 3 or 4, wherein the coffee beans are roasted at a temperature and for a time so as to achieve an average decrease of the total weight greater than 16.6 ± 1.2% and lower than 18.5 ± 1.2%.

11. The method according to claim 9 for preparing a roasted coffee according to claim 5 or 6, wherein the coffee beans are roasted at a temperature and for a time so as to achieve an average decrease of the total weight greater than or equal to 18.5 ± 1.2%.

12. A roasted coffee according to any of claims 1-6 for use in the prevention of type 2 diabetes.

**13.** The roasted coffee for use according to claim 12, wherein said coffee is taken in the form of a beverage.

**14.** The roasted coffee for use according to claim 13, wherein said beverage is prepared with a capsule according to claim 8.

**Patentansprüche**

**1.** Ein gerösteter Kaffee von *Coffea arabica* var. *Laurina,* **dadurch gekennzeichnet, dass** ein Pulver des genannten Kaffees Folgendes umfasst:

- 5,0 bis 10,0 mg/g Trigonellin; und
- 3,0 bis 8,0 mg/g Mono-Caffeoylchinasäuren.

**2.** Der geröstete Kaffee gemäß Anspruch 1, worin das Pulver des genannten gerösteten Kaffees eine durchschnittliche Färbung von weniger als 91 ± 3 AU (willkürliche Einheiten) hat, gemessen mittels eines Colorette 3B Kolorimeters, hergestellt von Probat.

**3.** Der geröstete Kaffee gemäß Anspruch 1, worin das Pulver Folgendes umfasst:

- 8,5 bis 10 mg/g Trigonellin; und
- 6,5 bis 8,0 mg/g Mono-Caffeoylchinasäuren.

**4.** Der geröstete Kaffee gemäß Anspruch 3, worin das Pulver des genannten gerösteten Kaffees eine durchschnittliche Färbung zwischen 62 ± 3 AU und 82 ± 3 AU hat, gemessen mittels eines Colorette 3B Kolorimeters, hergestellt von Probat.

**5.** Der geröstete Kaffee gemäß Anspruch 1, worin das Pulver Folgendes umfasst:

- 5,0 bis 6,5 mg/g Trigonellin; und
- 3,0 bis 4,5 mg/g Mono-Caffeoylchinasäuren.

**6.** Der geröstete Kaffee gemäß Anspruch 5, worin das Pulver des genannten gerösteten Kaffees eine durchschnittliche Färbung von weniger als oder gleich 62 ± 3 AU hat, gemessen mittels eines Colorette 3B Kolorimeters, hergestellt von Probat.

**7.** Ein Kaffeegetränk, hergestellt mit einem gerösteten Kaffee gemäß irgendeinem der vorhergehenden Ansprüche.

**8.** Eine Kapsel zum Extrahieren eines Getränks aus einer pulverisierten Substanz mittels Wasser unter Druck, wobei die genannte Kapsel **dadurch gekennzeichnet ist, dass** die pulverisierte Substanz ein Pulver von geröstetem Kaffee gemäß irgendeinem der Ansprüche 1-6 ist.

**9.** Ein Verfahren zur Herstellung eines gerösteten Kaffees gemäß irgendeinem der Ansprüche 1-6, **dadurch gekennzeichnet, dass** das genannte Verfahren den Schritt Rösten von grünen Kaffeebohnen von *Coffea arabica* var. *Laurina* bei einer Temperatur und für einen Zeitraum, um eine durchschnittliche Abnahme des Gesamtgewichts der Kaffeebohnen von mehr als 16,6 ± 1,2% zu erreichen, umfasst.

**10.** Das Verfahren gemäß Anspruch 9 zur Herstellung eines gerösteten Kaffees gemäß Anspruch 3 oder 4, worin die Kaffeebohnen bei einer Temperatur und für einen Zeitraum geröstet werden, um eine durchschnittliche Abnahme des Gesamtgewichts von mehr als 16,6 ± 1,2% und weniger als 18,5 ± 1,2% zu erreichen.

**11.** Das Verfahren gemäß Anspruch 9, zur Herstellung eines gerösteten Kaffees gemäß Anspruch 5 oder 6, worin die Kaffeebohnen bei einer Temperatur und für einen Zeitraum geröstet werden, um eine durchschnittliche Abnahme des Gesamtgewichts von mehr als oder gleich 18,5 ± 1,2% zu erreichen.

**12.** Ein gerösteter Kaffee gemäß irgendeinem der Ansprüche 1-6, zur Verwendung in der Prävention von Diabetes Typ 2.

**13.** Der geröstete Kaffee zur Verwendung gemäß Anspruch 12, worin der genannte Kaffee in der Form eines Getränks eingenommen wird.

**14.** Der geröstete Kaffee zur Verwendung gemäß Anspruch 13, worin das Getränk mit einer Kapsel gemäß Anspruch 8 hergestellt wird.

**Revendications**

**1.** Café torréfié de *Coffea arabica* var. *Laurina*, **caractérisé en ce qu'**une poudre dudit café comporte :

- 5,0 à 10,0 mg/g de trigonelline ; et
- 3,0 à 8,0 mg/g d'acides mono-cafféoylquiniques.

**2.** Café torréfié selon la revendication 1, dans lequel la poudre dudit café torréfié a une couleur moyenne inférieure à 91 ± 3 AU (unités arbitraires), mesurée au moyen d'un colorimètre Colorette 3B fabriqué par Probat.

**3.** Café torréfié selon la revendication 1, dans lequel la poudre comporte :

- 8,5 à 10,0 mg/g de trigonelline ; et
- 6,5 à 8,0 mg/g d'acides mono-cafféoylquiniques.

**4.** Café torréfié selon la revendication 3, dans lequel la poudre dudit café torréfié a une couleur moyenne comprise entre 62 ± 3 AU et 82 ± 3 AU, mesuré au moyen d'un colorimètre Colorette 3B fabriqué par Probat.

**5.** Café torréfié selon la revendication 1, dans lequel la poudre comporte :

- 5,0 à 6,5 mg/g de trigonelline ; et
- 3,0 à 4,5 mg/g d'acides mono-cafféoylquiniques.

**6.** Café torréfié selon la revendication 5, dans lequel la poudre dudit café torréfié a une couleur moyenne inférieure ou égale à 62 ± 3 AU, mesurée au moyen d'un colorimètre Colorette 3B fabriqué par Probat.

**7.** Boisson de café préparée avec un café torréfié selon l'une quelconque des revendications précédentes.

**8.** Capsule pour extraire une boisson d'une substance en poudre au moyen d'eau mise sous pression, ladite capsule étant **caractérisée en ce que** la substance en poudre est une poudre de café torréfié selon l'une quelconque des revendications 1 à 6.

**9.** Procédé pour préparer un café torréfié selon l'une quelconque des revendications 1 à 6, **caractérisé en ce que** ledit procédé comporte l'étape consistant à torréfier des grains de café vert de *Coffea arabica* var. *Laurina* à une certaine température et pendant une certaine durée de manière à obtenir une diminution moyenne du poids total des grains de café supérieure à 16,6 ± 1,2 %.

**10.** Procédé selon la revendication 9 pour préparer un café torréfié selon la revendication 3 ou 4, dans lequel les grains de café sont torréfiés à une certaine température et pendant une certaine durée de manière à obtenir une diminution moyenne du poids total supérieure à 16,6 ± 1,2 % et inférieure à 18,5 ± 1,2 %.

**11.** Procédé selon la revendication 9 pour préparer un café torréfié selon la revendication 5 ou 6, dans lequel les grains de café sont torréfiés à une certaine température et pendant une certaine durée de manière à obtenir une diminution moyenne du poids total supérieure ou égale à 18,5 ± 1,2 %.

**12.** Café torréfié selon l'une quelconque des revendications 1 à 6 pour une utilisation dans la prévention de diabètes de type 2.

**13.** Café torréfié pour une utilisation selon la revendication 12, dans lequel ledit café est pris sous la forme d'une boisson.

**14.** Café torréfié pour une utilisation selon la revendication 13, dans lequel ladite boisson est préparée avec une capsule selon la revendication 8.

*Fig. 1*

*Fig. 2*

*Fig.3*

*Fig.4*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100112098 A
- WO 2011134656 A
- WO 2008011913 A
- IT MI20130906 A

**Non-patent literature cited in the description**

- **HUXLEY et al.** British Medical Journal. 2006, vol. 332, 73-78
- **MORRISH et al.** *Diabetologia,* 2001, vol. 44 (2), 14-21
- **TUOMILEHTO et al.** *JAMA,* 2004, vol. 291, 1213-1219
- **SARTORELLI et al.** *Am J Clin Nutr.,* 2010, vol. 91, 1002-1012
- **HUXLEY et al.** *Arch Intern Med.,* 2009, vol. 169, 2053-2063
- **HALVORSEN et al.** *Am J Clin Nutr.,* 2006, vol. 84, 95-135
- **PELLEGRINI et al.** *J Nutr.,* 2003, vol. 133, 2812-2819
- **SCHULZE et al.** *Am J Clin Nutr.,* 2005, vol. 82, 675-684
- **LOPEZ-GARCIA et al.** *Am J Clin Nutr.,* 2006, vol. 84, 888-893
- **ARSENAULT et al.** *Eur J Clin Nutr.,* 2009, vol. 63, 1419-1424